# EUROPEAN PATENT APPLICATION

(11) **EP 1 738 695 A1**
(43) Date of publication of application: **03.01.2007**
(21) Application number: 06253248.6
(22) Date of filing: 22.06.2006
(51) Int. Cl.: A61B 17/12, B29C 70/70, A61L 31/08, A61L 31/14

(54) **Method of making a coated embolization device**

(30) Priority: 30.06.2005 US 172355
(71) Applicant: Cordis Development Corporation, Miami Lakes, FL 33014 (US)
(72) Inventor: Vyakarnam, Murty, South Orange, New Jersey 07079 (US); Do, Hiep Q., Hillsborough, New Jersey 08844 (US); Li, Yufu, Bridgewater, New Jersey 08807 (US); Yi, Chin-Feng, Hillsborough, New Jersey 08844-1133 (US)
(74) Representative: Belcher, Simon James

(57) **Abstract**

A vascular embolization device comprises an elongated coil having a lumen, said coil and lumen being at least partially embedded in an elongated foam member comprising a flexible, biodegradable, water insoluble, open, interconnecting-cell foam material having embolic characteristics, and capable of allowing cell proliferation into the open cell foam interior. A manufacturing method is disclosed. In one embodiment, the method comprises inserting an uncoated, vascular embolization coil into an inert tubing; filling the tubing with a solution of a coating material for the coil; freezing the solution to form a multiple phase, frozen mass in the tubing; and lyophilizing the frozen mass in the tubing to remove solvent therefrom, forming a porous coating on said coil.

## Description

Elongated coil embolization devices are well known in their use to embolize a blood vessel or a defect in a blood vessel such as an aneurysm or fistula, for example a brain aneurysm.

These embolic coils are of small diameter, on the order of 0.305 mm (0.012 inch), for example, to have the capability of fitting into small blood vessels. Embolic coils have great advantage over neurosurgical clipping, and are quickly becoming the desired standard of care.

In the prior art, embolic coils are placed into an aneurysm, which causes clotting to take place in the aneurysm, reducing the risk of aneurysm rupture. The aneurysm becomes protected by the clot, which occupies the volume of the aneurysm. However, a large number of aneurysms tend to recanalize with pressurized blood over time, which once again elevates the risk of aneurysm rupture, or growth of the aneurysm in size, along with causing compaction of the coils.

There is a need for a better, bioactive embolization device. The device of this invention is very flexible to ensure good packing. The device also enhances protein binding and cell attachment, so that the ingrowth of tissue into the aneurysm is encouraged, providing by the presence of living tissue a long-term solution to the problem of recanalization of an aneurysm.

By this invention, a vascular embolization device comprises an elongated coil having a lumen. The coil is at least partially embedded in an open interconnecting cell foam made from synthetic or natural polymer. More specifically, the coil is at least partially embedded in an elongated foam member comprising a flexible, biodegradable, water insoluble, open and interconnecting cell foam material having embolic characteristics, and capable of allowing cell proliferation into the cell foam interior. The elongated, flexible foam material typically has an uncompressed outer diameter that is no more than about 0.153 mm (0.006 inch) greater than the outer diameter of the elongated coil. Preferably, this uncompressed, outer diameter of the elongated foam member is no more than about 0.102 mm (0.004 inch) greater than the elongated coil outer diameter. Some or all of the lumen of the coil may also be filled with the foam material.

Thus, by this invention, the vascular embolization device with the embedded, elongated coil remains capable of sliding through appropriate catheters for implantation into a desired position in the vascular system of a patient, while at the same time a substantial surface area of foam material having embolic characteristics is provided. As stated, the foam is capable of allowing cell proliferation into the foam interior, so that the embolization device has sufficient cell adhering surface area to be effective, while being capable of sliding through a catheter that is little or no larger than the catheter used to place uncoated embolization coils. Preferably, the uncompressed, outer diameter of the foam member is no more than about 0.406 mm (0.016 inch), while the outer coil diameter, embedded therein, is about 0.254 to 0.356 mm (0.01 to 0.014 inch). Typically, the uncompressed, outer diameter of the foam member is no more than 25 percent greater than the coil outer diameter, while the coil lumen is substantially filled with the foam member. While the aforementioned configuration represents some preferred dimensions, in accordance with other embodiments of the present invention, coils may range in outer diameter from about 0.051 mm (0.002 inch) to about 3.81 mm (0.150 inch) and the outer diameter of the foam member may range from 25 percent to 1000 percent greater than the coil outer diameter.

In some embodiments, the foam member comprises a polymer formulation that can be both synthetic and/or naturally derived and that is flexible and cohesive, with or without the presence of water: i.e., it is not a hydrogel, that must be hydrated to be desirably soft and flexible. Specifically, the foam member may be elastomeric, and may comprise a variety of different, biodegradable, water insoluble, open and interconnecting-cell foams having embolic characteristics.

Biodegradable polymers readily break down into small segments when exposed to moist body tissue. The segments then either are absorbed by the body, or passed by the body. More particularly, the biodegraded segments do not elicit permanent, chronic foreign body reaction, because they are absorbed by the body or passed from the body, such that no permanent trace or residual of the segment is retained by the body.

The biodegradable polymers that can be used according to the present invention include conventional biocompatible, biodegradable polymers including polymers selected from the group consisting of aliphatic polyesters, poly(amino acids), copoly(ether-esters), polyalkylene oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, poly(propylene fumarate), poly(ester urethane), biomolecules (i.e., biopolymers such as collagen, elastin, biodegradable starches etc.) polyglycolides, polylactides, polylactones and blends thereof.

As used herein, the term "polyglycolide" is understood to include polyglycolic acid. Further, the term "polylactide" is understood to include polymers of L-lactide, D-lactide, meso-lactide, blends thereof, and lactic acid polymers and copolymers in which other moieties are present in amounts less than 50 mole percent.

Currently, aliphatic polyesters are among the biodegradable polymers for use in making the foam portion of the foam implants according to the present invention. Aliphatic polyesters can be homopolymers, copolymers (random, block, segmented, tapered blocks, graft, triblock, etc.) having a linear, branched, or star structure. Suitable monomers for making aliphatic homopolymers and copolymers may be selected from the group consisting of, but are not limited, to lactic acid (both L- and D-isomers), lactide (including L-, D-, and meso-lactide), glycolic acid, glycolide, epsilon-caprolactone, p-dioxanone (1,4-dioxan-2-one), trimethylene carbonate (1,3-dioxan-2-one), and combinations thereof.

Elastomeric copolymers are also particularly useful in the present invention. Suitable elastomeric polymers include those with an inherent viscosity of 0.8 dL/gram or greater, in some embodiments about 1.2 dL/gram to 4 dL/gram and in other embodiments about 1.4 dL/gram to 2 dL/gram, as determined at 25° C. in a 0.1 gram per deciliter (gram/dL) solution of polymer in hexafluoroisopropanol (HFIP). Further, suitable elastomers exhibit a high percent elongation and a low modulus, while possessing good tensile strength and good recovery characteristics. In one embodiment of this invention, the elastomer from which the foam component is formed exhibits a percent elongation (e.g., greater than about 200 percent and sometimes greater than about 500 percent). In addition to these elongation and modulus properties, suitable elastomers should also have a tensile strength greater than about 3.45 MPa (500 psi), sometimes greater than about 6.89 MPa (1,000 psi), and a tear strength of greater than about 0.895 kg/mm (50 lbs/inch), or even greater than about 1.432 kg/mm (80 lbs/inch).

Exemplary biodegradable, biocompatible elastomers include but are not limited to elastomeric copolymers of epsilon-caprolactone and glycolide (including polyglycolic acid) with a mole ratio of epsilon-caprolactone to glycolide of from about 35/65 to about 65/35, or from 45/55 to 35/65; elastomeric copolymers of epsilon-caprolactone and lactide (including L-lactide, D-lactide, blends thereof, and lactic acid polymers and copolymers) where the mole ratio of epsilon-caprolactone to lactide is from about 30/70 to about 95/5 and in some embodiments from 30/70 to 45/55 or from about 85/15 to about 95/5; elastomeric copolymers of p-dioxanone (1, 4-dioxan-2-one) and lactide (including L-lactide, D-lactide, blends thereof, and lactic acid polymers and copolymers) where the mole ratio of p-dioxanone to lactide is from about 40/60 to about 60/40; elastomeric copolymers of epsilon-caprolactone and p-dioxanone where the mole ratio of epsilon-caprolactone to p-dioxanone is from about from 30/70 to about 70/30; elastomeric copolymers of p-dioxanone and trimethylene carbonate where the mole ratio of p-dioxanone to trimethylene carbonate is from about 30/70 to about 70/30; elastomeric copolymers of trimethylene carbonate and glycolide (including polyglycolic acid) where the mole ratio of trimethylene carbonate to glycolide is from about 30/70 to about 70/30; elastomeric copolymers of trimethylene carbonate and lactide (including L-lactide, D-lactide, blends thereof, and lactic acid polymers and copolymers) where the mole ratio of trimethylene carbonate to lactide is from about 30/70 to about 70/30); and blends thereof. It is to be understood that the exemplary biodegradable, biocompatible elastomers may be generally synthesized by a ring-opening polymerization of the corresponding lactone monomers or by polycondensation of the corresponding hydroxy-acids, or by combinations of these two polymerization methodologies.

One of ordinary skill in the art will appreciate that the selection of a suitable polymer or copolymer for forming the foam depends on several factors. The more relevant factors in the selection of the appropriate polymer(s) that is used to form the foam component include biodegradation kinetics; in-vivo mechanical performance; cell response to the material in terms of cell attachment, proliferation, migration and differentiation; and biocompatibility. Other relevant factors, which to some extent dictate the in-vitro and in-vivo behavior of the polymer, include the chemical composition, spatial distribution of the constituents, the molecular weight of the polymer, and the degree of crystallinity.

The ability of the material substrate to biodegrade in a timely fashion in the body environment is also significant. But the differences in the biodegradation time under in-vivo conditions can also be the basis for combining two different copolymers. For example, a copolymer of 35/65 epsilon-caprolactone and glycolide (a relatively fast biodegrading polymer) is blended with 40/60 epsilon-caprolactone and L-lactide copolymer (a relatively slow biodegrading polymer) to form a foam component. Depending upon the processing technique used, the two constituents can be either randomly inter-connected bicontinuous phases, or the constituents could have a gradient-like architecture in the form of a laminate type composite with a well integrated interface between the two constituent layers. The microstructure of these foams can be optimized for the required application.

In one embodiment it is desirable to use polymer blends to form structures which transition from one composition to another composition in a gradient-like architecture. For example, by blending an elastomer of epsilon-caprolactone-co-glycolide with epsilon-caprolactone-co-lactide (e.g., with a mole ratio of about 5/95), a foam may be formed that transitions from a softer, spongy material to a stiffer, more rigid material. Clearly, one of ordinary skill in the art will appreciate that other polymer blends may be used for similar gradient effects, or to provide different gradients (e.g., different biodegradation profiles, stress response profiles, or different degrees of elasticity).

Methods for manufacturing the foam component of the current device include a variety of methods known and used in this art. For example, they include lyophilization, supercritical solvent foaming, extrusion or mold foaming (e.g. external gas injection or in situ gas generation), casting with an extractable material (e.g., salts, sugar or similar suitable materials) and the like.

Of particular utility is foam formation in accordance with this invention by freeze drying or lyophilization. The advantages of lyophilization include the avoidance of elevated temperatures, thus minimizing the potential for temperature-associated degradation and enabling the inclusion of temperature sensitive bioactive agents. Additional advantages include the ability to control the pore size and porosity of the foamed material. Non-aqueous lyophilization also eliminates the need for exposure of the processed material to water as is required in salt leaching processes that may cause premature hydrolysis. Lyophylization is a cost effective, simple, one-step process that facilitates manufacturing, and is widely known and used in the food and pharmaceutical industries.

Lyophilization is a process for removing a (frozen or crystallized) solvent, frequently water, from various materials. Lyophilization of enzymes, antibodies, and sensitive biological materials is quite often the method of choice for extending the shelf life of these products and preserving their biological activity. As practiced as a means of foam formation, the lyophilization process usually requires that a polymeric material be rendered soluble in a crystallizable solvent capable of being sublimed, usually at reduced pressure. Although the solvent may be water, other solvents such as 1,4-dioxane may be used. This latter solvent has found great utility in foam formation because many medically important polymers are soluble in it. It is crystallizable (melting point approximately 12° C.), and it can generate a significant vapor pressure at temperatures in which it is still a solid, i.e. it can be sublimed at reduced pressure.

While portions only of the entire length of the elongated coil may be embedded in separate lengths of the elongated foam member, in other embodiments, the entire length of the elongated coil is embedded in the elongated foam member.

The elongated coil may be made of conventional materials for use as a vascular embolization device, for example, platinum, gold, tungsten, titanium, or suitable alloys thereof.

In some embodiments, the elongated foam member has an average pore size of 5-500 µm (5-500 microns), which permits the desired cell proliferation into the open cell foam interior.

The porous foam member in which the elongated coil is embedded is desirably elastomeric (i.e. having an elongation to break of at least 100%) to permit compression of the body within a catheter, although in some embodiments only a relatively minor amount of the mass of the elongated foam member is outside of the coil and, thus, needing compression when placed in the catheter.

Also, a flexible, elastomeric material is less likely to chip off, thus avoiding fragments that can form emboli elsewhere in the body, which is of course highly undesirable.

By this means, an easily deployable, vascular embolization device is provided which has less tendency to encounter the subsequent problem of recanalization, since the porous foam increases protein binding and cell attachment to encourage tissue ingrowth, which is a long-term solution to the recanalization problem. By this invention, tissue response can be modulated in such a way that, rather than triggering a severe thrombolitic response, protein binding and cell attachment is enhanced. A mild to moderate inflammatory response can be induced that promotes organization of fibrotic tissue, leading to the desired tissue ingrowth. Also, the packing density of the embolization devices of this invention can be higher than uncoated coils of the prior art, since the porous layer expands as it exits from the catheter, forming its natural, unstressed size, with a diameter which is slightly larger than the coils embedded in them. Thus, more of the aneurysm is filled, resulting in more blood stagnation for forming the initial clot, and compensating for the natural contours of an aneurysm shape, to provide better outcomes.

Preferably, the coating composition of this invention comprises a known, elastomeric, foamed copolymer of epsilon caprolactone and glycolide (referred to hereafter as PCL/PGA). Unlike collagen-based coatings, the PCL/PGA coating is not pro-thrombolitic, thereby allowing the surgeon to manipulate the precise location of the embolization device for deployment. Furthermore, the PCL/PGA coating can provide consistent deployment, not significantly altering the physical characteristics of the coil. The degree of pro-inflammatory response can be controlled by changing the amount and formulation of the polymer coated on the coil, changing the nature of the composition, or adding of low molecular weight glycolide or glycolic acid monomers.

The elongated coils used herein are known to the art and are generally metallic, being introduced into a neurovascular space, for example, using an image guided system. The coils are preformed during manufacturing to attain random or helical shapes that easily conform to a space such as an aneurysm. Once deployed at the proper location within the vascular system of the patient, they are detached from the delivery system, such as a catheter, by conventional means. The coils generally retain their pre-formed shapes, to ensure integrity of the filling of the aneurysm or the like, and to reduce the chance of migration of the coil from the aneurysm sac into the vasculature.

The foamed, porous polymers may be created by known physical or chemical means. However, in accordance with this invention, a method of making a porous, vascular embolization device with an embedded, elongated coil comprises the steps of: creating a generally homogeneous solution (which includes dispersions such as colloids) of the desired polymer from which the foam member is made; placing the coil in a tubular mold and filling the mold with the polymer solution; cooling the mold at a desired cooling rate for the polymer solution to freeze and separate into phases; and vacuum drying the frozen polymer solution, to remove the solvent essentially by sublimation, thus forming the elongated foam member with the coil embedded therein.

An annealing step may also be used if desired, to provide a solvent-free, stable, porous, elongated foam member with the coil embedded therein, and which has a long shelf life. Porosity (pore density), pore size, and surface area of the elongated foam member can be controlled by process variables such as polymer solution concentration, the particular polymer-solvent system used, the cooling rate, and a ratio of the coil outer diameter to the inner diameter of the tube in which the coil resides during the process.

Other methods of creating porous coils can include the introduction of a fugitive compound, such as a soluble salt, that is not actually a solvent, but can be admixed into the polymer solution or polymer melt, and, once the device is coated or filled into a mold and subsequently dried or cooled, the fugitive compound can be removed by dissolution in a suitable solvent, such as water. Such materials are deemed to be a "solvent" herein for the purpose of defining the invention.

Alternatively, the fugitive compound can be a blowing agent for creation of the foam by forming bubbles.

Furthermore, the porous polymer of the elongated foam member can act as a depot for controlled release of a therapeutic agent. For example, a chemotactic agent can be present to induce cellular activity.

In another aspect of the invention, a method is provided for making a coated, vascular embolization device, which comprises: inserting an uncoated, vascular embolization coil into an inert, typically plastic tubing (which acts as a mold); filing the tubing with a generally homogeneous solution of a coating material for the coil; freezing the solution to form a multiple-phase, frozen mass in the tubing; and lyophilizing the frozen mass in the tubing to remove solvent therefrom, forming a porous coating on said coil. The coating formed may be an open cell foam coating, in currently preferred embodiments, for the advantages stated above.

In some embodiments, the inner plastic tubing may have an inner diameter of about 0.330 to 0.813 mm (0.013 to 0.032 inch). The vascular embolization coil may have a diameter of about 0.254 to 0.508 (0.01 to 0.02 inch).

The coating material may, in currently preferred embodiments, consist essentially of a PCL/PGA copolymer.

The solution contained in the tubing may, in the step of freezing or cooling, be frozen in ice water, a dry ice/methanol solution, liquid nitrogen or another cryogenic fluid. As above, the elongated coil may be made of the normal materials used for an embolic coil, such as platinum and others listed above.

Embodiments of the invention will now be described by way of example with reference to the accompanying drawings, in which:
Fig. 1 is a fragmentary, enlarged view of an embolic coil in accordance with this invention, with portions broken away.
Fig. 2 is a diagrammatic view of a process for steps of manufacturing the vascular embolization device of Fig. 1.

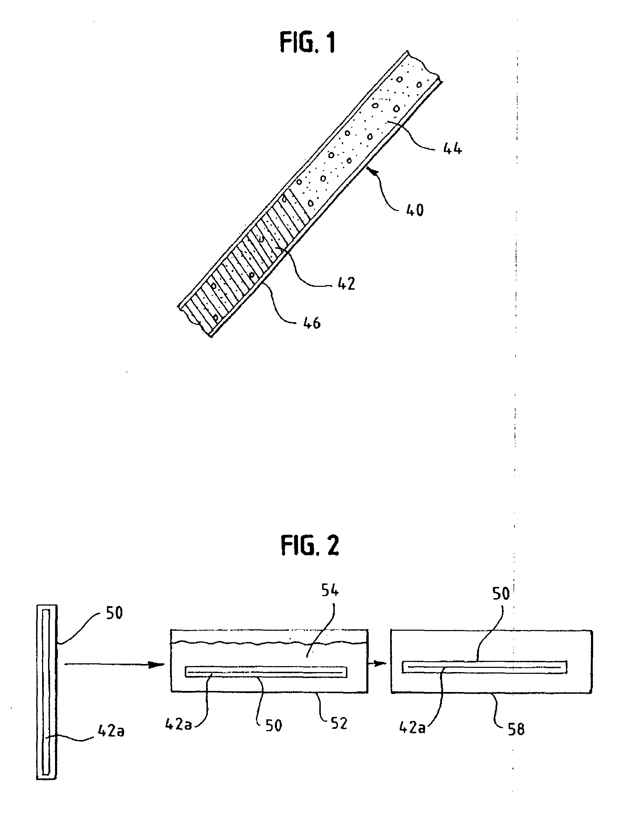

Referring to the drawings, Fig. 1 shows a vascular embolization device 10, which comprises a helical embolic coil 12, which may be made of platinum or the like, and may be of conventional dimensions for an embolic coil, for example, an outer diameter of 0.305 mm (0.012 inch). Such an embolic coil can be used in the treatment of brain aneurysms.

Only a portion of embolic coil 12 is shown. A typical embolic coil may be on the order of 10 cm. in length, and may naturally bunch up into a clump of material, to facilitate its retention in an aneurysm sack or the like. While the vascular embolization devices shown in the drawings are straight, in fact, they tend to naturally assume a non-straight configuration, of a form as desired for the particular treatment.

Elongated coil 12 is shown to be embedded in an elongated foam member 14, which occupies the lumen of coil 12, and also typically extends outwardly between the coils of coil 12 to form a relatively thin, outer coating 16 of the same foam material as is found in the inner foam portion 14 that is occupying the lumen of coil 12. In this embodiment, the thickness of outer foam layer 16 is about 0.025 mm (0.001 inch), surrounding coil 12 on all sides, and connected with the internal foam body 14. Thus, such an embolization device is capable of sliding in a catheter, compressing the generally cylindrical foam layer 16, without creating an undue amount of friction in a catheter, which catheter is only slightly enlarged in its inner diameter, or not enlarged at all, over a catheter that is used for the emplacement of a bare embolic coil similar to coil 12. A portion of coil 12 has been omitted in Fig. 1, to better illustrate the inner foam body 14 present in the lumen of coil 12, but, in reality, coil 12 extends the entire length of Fig. 1.

Foam body 14, 16 may be an open cell, porous foam made of a copolymer of about 36 weight percent PCL and 64 weight percent PGA, which is a known material for coating of embolic coils. Other proportions of PCL to PGA may also be used. The open cell pores of the material used may have an average pore size of 5-500 microns, providing an embolization device into which cells of the body can migrate, and proliferate through the network of open cell pores of the foam into the interior thereof. The solid foam material forms a bed to receive and carry the proliferating cells. Furthermore, the foam is biodegradable, permitting the slow replacement of the foam material by proliferating tissue, the result of this being that, over time, tissue proliferates throughout the aneurysm sac, resulting in a more stable securance of the aneurysm, with reduced possibility of recanalization and the return of a dangerous condition with respect to the aneurysm.

When compared with a bare metal embolization coil, embolization devices similar to that of Fig. 1 can exhibit a significant increase in fibroblast cell adhesion over a two-hour period, on the order of 33% greater. Also, there can be increase of fibronectin binding on the embolization device similar to that of Fig. 1, showing a three-fold increase or more over a corresponding, bare metal embolization coil.

These improvements result in better cell proliferation through the vascular embolization devices of this invention, when compared with corresponding bare metal embolic coils.

Turning to Fig. 2, a graphic flow chart is shown of steps used in a method for manufacturing embolization devices in accordance with this invention. A 10 cm length of a platinum embolic coil 12a was inserted into 12 cm of polytetrafluoroethylene (PTFE) tubing 20, having an inner diameter of 0.559 mm (0.022 inch). While Fig. 2 shows the embolic coil 12a to be straight, in fact it has many curves and kinks, naturally folding up into a little bundle or wad, and thus would not be precisely straight as retained in tube 20. In one embodiment, the resulting tube 20 containing coil 12a is filled with a solution of 5% (w/w) of 36% PCL/64% PGA copolymer, dissolved in 1,4-dioxane from Fisher Scientific Company. When the lumen of tube 20 is completely filled, the tube is immersed into container 22 containing liquid nitrogen 24 for five minutes. Upon the freezing of the solution with liquid nitrogen, there is a phase separation between the PCL/PGA copolymer and the 1,4-dioxane. Then, the tubing 20 containing coil 12a and frozen solution is placed into an aluminum mold or open metal rack, dish or plate 28, which has been pre-cooled to - 17°C. The aluminum mold, rack, dish or plate is stored in a lyophilization (freeze-drying) chamber, under vacuum, so that the 1,4-dioxane evaporates out of the lumen of tubing 20, leaving the PCL/PGA polymer as a porous foam, carried inside and outside of embolic coil 12a. The resulting mass of PCL/PCA in which coil 12a is embedded comprises an open-cell foam.

The above disclosure, and the examples below, are for illustrative purposes only, and are not to limit the scope of the invention, which is as described in the claims below.

### Example 1: Polymer solution Preparation:

A 1 % w/w solution of epsilon-caprolactone/glycolide copolymer in 1,4-dioxane was prepared as follows. 1.5 grams of 36/64 poly(epsilon-caprolactone-glycolic acid) copolymer, obtained from American Polymer Incorporation (American Polymer Inc., Birmingham, AL) was added to 148.8 grams of 1,4-dioxane (Fisher Scientific, Raritan, NJ) in a 250 milliliter Erlenmeyer screwed cap flask. The mixture was stirred for 4 hours in 60°C water bath set on a temperature controlled heating plate. The polymer solution was filtered through an extra coarse thimble filter to remove any undissolved solids.

### Example 2: Making 36/64 PCL/PGA Foamed coils using freeze-dry method.

A 10-cm embolic coil was inserted in a polytetrafluoroethylene (PTFE) tubing (0.015" ID, ZEUS Industrial products, Inc. Orangeburg, SC) that was 2 centimeters longer that the coil. The tube served as a mold. The tube mold containing the coil was filled with the 1% w/w solution of the epsilon-caprolactone/glycolide copolymer in 1,4-dioxane described in Example 1, using a glass syringe with a 30G needle attached. The tube mold containing the coil and solution was sonicated for approximately 5 minutes in a sonication bath (BRANSON, Model 3210R-DTH, Danbury, CT) to ensure the solution got into the inter-diameter of the coil, between the coils and into the coil lumen. Bubbles generated during sonication were replaced with fresh polymer solution. Then, the tube mold was frozen in liquid nitrogen for about five minutes, and placed on a pre-cooled (-17°C) shelf of a freeze-dryer (lyophilization unit) (FTS Systems, Model TD3B2T5100, Stone Ridge, NY) The following freeze-drying cycle was the used:

| **Temp(°)** | **Pressure (mtor)** | **Hold time (min)** |
|---|---|---|
| -17 | Ambient pressure | 15 |
| -17 | 100 | 120 |
| -5 | 20 | 120 |
| +5 | 20 | 60 |
| 20 | 20 | 60 |

### Example 3: Fibronectin Absorption Experiment

A 4-cm foam-embedded coil from Example 2 was cut into about 4-mm segments. Coil segments were then placed in to Siliconized Eppendorf tubes. The coil segments were wet with 0.5 milliliters of 70% ethanol, and then washed twice with 1 milliliter of phosphate buffered saline (PBS). The coil segments were incubated with 100 ml of fibronectin probe solution. The solution was purchased from Sigma Chemical Co. (St. Louis, MO). and was radio labeled by iodine 125 at Lofstrand Labs Limited (Gaithersburg, MD). The incubation was for 1 hour at 37 °C. Afterwards, the incubated coil segments were washed three times with 1 milliliter 0.1% Triton in PBS. The coil segments were transferred to a scintillation vial and the radioactivity of the samples was counted. Absorption of fibronectin on foamed coils of Example 2 was about 3 fold higher than on the bare coils under similar treatment.

## Claims

1. A method of making a coated, vascular embolization device, the method comprising:
inserting an uncoated, vascular embolization coil into an inert tubing;
filling the tubing with a solution of a coating material for the coil;
freezing the solution to form a multiple phase, frozen mass in the tubing; and
lyophilizing the frozen mass in the tubing to remove solvent therefrom, forming a porous coating on said coil.

2. The method of claim 1 in which the coating form is an open cell foam coating.

3. The method of claim 1 in which the inert plastic tubing has an inner diameter of about 0.381 to 0.762 mm (0.015 to 0.03 inch), and the vascular embolization coil has a diameter of about 0.254 to 0.508 mm (0.01 to 0.02 inch).

4. The method of claim 1 in which the coating materials consists essentially a polymer that is synthetic or naturally derived.

5. The method of claim 1 in which the coating material comprises a PCL/PGA copolymer, and in which the coating formed is an open cell foam coating.

6. The method of claim 5 in which the inert tubing is PTFE tubing.

7. The method of claim 5 in which the inert tubing has an inner diameter of about 0.330 to 0.813 mm (0.013 to 0.032 inch), and the vascular embolization coil has a diameter of about 0.254 to 0.508 mm (0.01 to 0.02 inch).

8. The method of claim 1 or 7 in which the solution is frozen in liquid nitrogen in the freezing step.

9. The method of claim 1 or 8 in which said coil comprises platinum.
